# EUROPÄISCHE PATENTSCHRIFT

(11) **EP 1 278 834 B1**
(45) Veröffentlichungstag und Bekanntmachung des Hinweises auf die Patenterteilung: **28.12.2005**
(21) Anmeldenummer: 01931377.4
(22) Anmeldetag: 22.03.2001
(51) Int. Cl.: C12N 15/10, B01J 43/00, B01J 19/00

(54) **VERFAHREN FÜR DIE KOMPLEXE NUKLEINSÄUREANALYTIK**
PROCESS FOR COMPLEX NUCLEIC ACID ANALYSIS
METHODE POUR L'ANALYSE COMPLEXE D'ACIDES NUCLEIQUES

(30) Priorität: 22.03.2000 DE 10013990
(43) Veröffentlichungstag der Anmeldung: 29.01.2003
(73) Patentinhaber: Invitek Gesellschaft für Biotechnik & Biodesign mbH., 13125 Berlin (DE); Poly-An Gesellschaft zur Herstellung von Polymeren für Spezielle Anwendungen und Analytik mbH, 13086 Berlin (DE)
(72) Erfinder: BENDZKO, Peter, 12623 Berlin (DE); SCHEDLER, Uwe, 10119 Berlin (DE); MATUSCHEWSKI, Heike, 13187 Berlin (DE); HILLEBRAND, Timo, 15366 Hönow (DE)
(74) Vertreter: Schneider, Henry
(86) Internationale Anmeldenummer: PCT/DE2001/001102
(87) Internationale Veröffentlichungsnummer: WO 2001/070386

(56) Entgegenhaltungen:
- WO-A-00/12575
- WO-A-00/27496
- DD-A- 280 698
- DE-A- 4 309 248
- US-A- 4 097 420
- KURGANOV A A ET AL: "ION-EXCHANGE HIGH-PERFORMANCE LIQUID CHROMATOGRAPHY OF NUCLEOTIDES AND POLYPEPTIDES ON NEW TYPES OF ION-EXCHANGE SORBENTS, BASED ON POLYSTYRENE-COATED SILICAS" JOURNAL OF CHROMATOGRAPHY, ELSEVIER SCIENCE PUBLISHERS B.V. AMSTERDAM, NL, Bd. 548, Nr. 1 / 2, 12. Juli 1991 (1991-07-12), Seiten 207-214, XP000218659 ISSN: 0021-9673
- SCHULZ M ET AL: "FUNKTIONALISIERTE POLYMEROBERFLAECHEN" CLB. CHEMIE IN LABOR UND BIOTECHNIK, VERLAG BREIDENSTEIN, FRANKFURT AM MAIN, DE, Bd. 51, Nr. 3, 2000, Seiten 84-86, XP001015413 ISSN: 0943-6677
- ULBRICHT M ET AL: "PHOTO-INDUCED GRAFT POLYMERIZATION SURFACE MODIFICATIONS FOR THE PREPARATION OF HYDROPHILIC AND LOW-PROTEIN-ADSORBING ULTRAFILTRATION MEMBRANES" JOURNAL OF MEMBRANE SCIENCE, ELSEVIER SCIENTIFIC PUBL.COMPANY. AMSTERDAM, NL, Bd. 115, Nr. 1, 26. Juni 1996 (1996-06-26), Seiten 31-47, XP000726906 ISSN: 0376-7388
- INTERNETAUSDRUCK ÜBER DAS PRODUKT "GENECLEAN" DER FIRMA BIO 101
- SAAL B.: DISSERTATION UNIVERSITÄT HANNOVER, 1998, SEITEN 10-11
- SUFFIA I. ET AL INFECTION AND IMMUNITY Bd. 68, Nr. 2, Februar 2000, Seiten 630 - 636
- VOGELSTEIN B.; GILLESPIE D.: 'PREPARATIVE AND ANALYTICAL PURIFICTAION OF DNA FROM AGAROSE' PNAS Bd. 76, Nr. 2, 01 Februar 1979, Seiten 615 - 619

## Beschreibung

Die Erfindung betrifft ein Verfahren zur komplexen und automatisierbaren Nukleinsäureanalytik.

Potentielle Anwendungsfelder betreffen eine Vielzahl von molekularbiologisch arbeitenden Laboratorien in den Bereichen der medizinischen Diagnostik, forensischen Diagnostik und Lebensmitteldiagnostik sowie alle anderen angrenzenden Forschungsgebiete.

Die Analyse von Nukleinsäuren (RNA und DNA) gewinnt immer stärker an Bedeutung. Dies betrifft alle Bereiche der molekularen biotechnologisch geprägten Forschung. Ein Schwerpunkt der molekularen Nukleinsäureanalytik in der medizinischen Forschung ist der Nachweis von Sequenzveränderungen in medizinisch interessanten Genen, welche in einem kausalen Zusammenhang mit pathologischen Prozessen betrachtet werden.
Auf Grund der großen Mengen an zu untersuchenden Targetsequenzen, setzen sich dabei mehr und mehr die "high-throughput-Anwendungen" (Hochdurchsatzapplikationen) durch. Unter den potentiellen Hochdurchsatzapplikationen finden dabei die auf DNA-Chips (DNA-arrays) basierenden Verfahrenslösungen mehr und mehr Einzug in die experimentelle Nukleinsäureanalytik.
Das Prinzip dieser Technologien besteht darin, dass eine unterschiedlich hohe Anzahl von Oligonukleotiden (Sonden) adressiert an feste Phasen gekoppelt bzw. de novo an festen Phasen synthetisiert wird. Die erzeugten DNA-Chips werden dann entsprechend jeweiliger Fragestellungen mit der zu analysierenden Probennukleinsäure inkubiert. Als zu analysierende Probennukleinsäure wurden bisher markierte total RNA zur Untersuchung von Expressionsprofilen sowie markierte *via* PCR erzeugte DNA-Fragmente zur Sequenzbestimmung (und Sequenzabweichung) eingesetzt (*US 5837832, US 6027880*).
Das bedeutet, dass jeweils entsprechend einer vorliegenden Fragestellung DNA-Chips mit spezifischen Oligonukleotidsonden belegt werden und nachfolgend mit der Probennukleinsäure hybridisiert werden. Das Hybridisierungsergebnis wird mittels Fluoreszenzdetektion ausgelesen und ausgewertet und liefert somit die detaillierte Information zur vorliegenden Nukleinsäuresequenz.
Mittels dieser Technologie ist es möglich, eine Vielzahl von Sequenzinformation zu analysieren. Nachteilig sind die extrem hohen Kosten sowie die Problematik einer exakten Qualitätskontrolle der auf einem Chip adressierten Oligonukleotidsonden bezüglich ihrer Sequenzidentität.

Ein weiterer großer Nachteil des Verfahrens besteht darin, dass für die Analyse von Nukleinsäuretargets hinsichtlich eines Sequenznachweises, die zu untersuchenden Targets zur Verfügung gestellt werden müssen. Diese Notwendigkeit schließt ein, aus einer relevanten Probe i.d.R. Nukleinsäure zu isolieren und nachfolgend die zu untersuchenden Targetsequenzen mittels PCR zu amplifizieren. Erst mit amplifizierten Targetsequenzen kann dann ein definiert mit Oligonukleotiden adressierter Chip hybridisiert werden.

Aus WO 00/12575 A sind Trägermaterialien bekannt, die aus einem polymeren Träger mit einer durch Pfropfcopolymerisation erhaltenen Beschichtung aus beliebig funktionalisierten Seitenketten bestehen. Die Trägermaterialien werden beispielsweise zur Identifizierung von Liganden (z.B. Bindung Nukleinsäuren) eingesetzt. Dafür wird durch parallele und kombinatorische Synthese eine Substanzbibliothek an dem Trägermaterial synthetisiert und die trägergebundenen Substanzen mit dem Ligand inkubiert, um diesen durch geeignete Methoden nachzuweisen.

Aus US 4,097,420 A und DD 280 698 sind Austauschermaterialien für die Aufreinigung biologischer Materialien, insbesondere Nukleinsäuren bekannt, wobei die Trägermaterialien gleichzeitig negative und positive funktionelle Gruppen enthalten können. Eine Bindung von Nukleinsäure an negative Gruppen wird nicht beschrieben.

Kurganov et al. (Ion-exchange high-performance liquid chromatography of nucleotides and polypeptides on new types of ion-exchange sorbents, based on polystyrene coated silicas, Journal of Chromatography (1991), 548, 207-214) beschreiben die Analyse eines Nukleotidgemischs, wobei die Nukleotide an positiv geladene Gruppen einer Beschichtung eines Trägermaterials (Anionenaustauscher) gebunden und anschließend mit einem Salzgradient eluiert werden.

Aus GB 2,274,409 A sind Membranen bekannt, die strukturierte negativ und positiv geladene Bereiche aufweisen. Das heißt, dass positiv und negativ geladene Areale vorhanden sind, die räumlich voneinander abgegrenzt sind. Solche Membranen werden als Trennmedien vielfältig in klassischen Filtrationsverfahren eingesetzt. Diese Membranen sind jedoch für einen komplexen Ablauf in der Nukleinsäuremanipulation und -analytik ungeeignet, da eine Reinigung, Bindung sowie Detektion der Nukleinsäuren aufgrund der räumlichen Trennung von positiv und negativ geladenen Gebieten nicht an einem Ort stattfinden kann.

Aufgabe der vorliegenden Erfindung war es deshalb, ein Verfahren für eine neuartige Plattformtechnologie zur komplexen Nukleinsäuremanipulation und Nukleinsäureanalytik bereitzustellen, das die Durchführung der relevanten Verfahrensschritte vereinfacht.

Dies wird erfindungsgemäß durch ein Verfahren gemäß Anspruch 1 erreicht, bei dem ein Trägermaterial verwendet wird, das auf seiner Oberfläche mindestens eine kovalent gebundene Schicht aufweist, welche mindestens zwei verschiedene funktionelle Gruppen trägt, die statistisch verteilt auf der Oberfläche vorliegen, wobei mindestens eine der funktionellen Gruppen negativ geladen ist und mindestens eine weitere funktionelle Gruppe positiv geladen und/oder chemisch reaktiv ist, und wobei
(a) ein die zu analysierende Nukleinsäure enthaltendes Probenmaterial mit dem Trägermaterial inkubiert wird, wobei die Nukleinsäure an der mindestens einen negativen funktionellen Gruppe des Trägermaterials gebunden wird,
(b) die zu analysierende Nukleinsäure von der negativen funktionellen Gruppe gelöst und kovalent an die positive und/oder chemisch reaktive funktionelle Gruppe des Trägermaterials gebunden wird und
(c) weitere für die Analytik notwendige Vorbereitungs- und/oder Analyseschritte gleichzeitig oder nacheinander an der an dem Trägermaterial gebundenen Nukleinsäure durchgeführt werden.
Die Unteransprüche beschreiben Vorzugsvarianten.

Durch das erfindungsgemäße Verfahren werden bisher verfolgte Chip-basierende Technologien von ihrem Funktionsprinzip umgekehrt. Durch die Verwendung einer bi- oder mehrfach funktionalisierten Oberfläche können alle bisher für eine Nukleinsäureanalytik (z.B. zur Sequenzbestimmung) notwendigen Prozesse an einem einzigen Träger realisiert werden, wodurch die Nukleinsäureanalytik insgesamt vereinfacht wird. Das erfindungsgemäße Verfahren ermöglicht insbesondere die komplexen Prozesse von Probenvorbereitung, ggf. spezifische Amplifikation, ggf. spezifische Manipulation und die zur Sequenzbestimmung notwendige Hybridisierungsreaktionen sowie finale Detektion räumlich fixiert an nur einer Reaktionsoberfläche ablaufen zu lassen. Das ist ein wesentlicher Vorteil gegenüber der bisher angewandten Technologie der komplexen Nukleinsäureanalytik, denn bei den bisherigen Chip-Verfahren laufen alle notwendigen Prozesse der Probenvorbereitung, Amplifikation und ggf. Manipulation immer räumlich getrennt ab.

Die Umkehrung der bisherigen Chip-Technologien basiert darauf, dass im Gegensatz zum Stand der Technik keine DNA-Chips generiert werden, sondern das erfindungsgemäße Mittel als feste Phase die zu untersuchende Probennukleinsäure immobilisiert.

Dies wird dadurch erreicht, dass eine klinisch relevante Probe mit einem Lysepuffersystem und der festen Phase inkubiert wird und nach erfolgter Lyse des Ausgangsmaterials ggf. unter Zusatz eines weiteren Puffers die Nukleinsäure der Zelle an die negativen funktionellen Gruppen der festen Phase gebunden wird. Danach wird die feste Phase mit der gebundenen Nukleinsäure gewaschen und potentielle Inhibitoren für nachfolgende Prozesse effektiv entfernt. Im weiteren Verfahrensablauf erfolgt nun die kovalente Immobilisierung der Probennukleinsäure durch Ablösung der Nukleinsäure von den negativen funktionellen Gruppen über die Inkubation der festen Phase mit einem Niedrigsalzpuffersystem (z.B. 10 mM Tris HCl). Dies hat zur Folge, dass die von der negativen Ladung abgetrennte Nukleinsäure ihrerseits auf Grund der vorliegenden Negativität mit den positiven funktionellen Gruppen der festen Phase in Wechselwirkung gelangen. Die letztlich erreichte kovalente Bindung erfolgt unter chemischer oder photochemischer Generierung von Radikalen auf der Oberfläche, die ihrerseits durch H-Abstraktion kovalente Bindungen knüpfen.
Die jetzt kovalent immobilisierte Nukleinsäure kann nachfolgend in beliebiger Form manipuliert werden. In einer Ausführungsvariante werden z.B. durch alkalische Denaturierung DNA-Einzelstränge erzeugt und über dem Fachmann bekannte Hybridisierungsverfahren mit spezifischen Oligonukleotidsonden ein Targetnachweis durchgeführt, wobei das Hybridisierungsergebnis mit nach dem Stand der Technik bekannten Verfahren bestimmt wird.
Durch den Einsatz von multiplen Sonden mit verschiedenen Nachweismarkern kann dann z.B. sehr einfach eine Vielzahl von Sequenzinformation bestimmt werden. Weiterhin besteht auch die Möglichkeit über hochstringente Waschschritte Sonden wieder zu entfernen, um nachfolgend das Verfahren zum Nachweis anderer Targetsequenzen in der Nukleinsäureprobe erneut zu beginnen.
Aus diesem Beispiel wird deutlich, dass prinzipiell jede Manipulation von Nukleinsäuren, die für eine Nukleinsäueanalytik interessant sind, mittels des erfindungsgemäßen Verfahrens realisiert werden kann. Durch die Integration aller Prozesse von der Probenvorbereitung (Nukleinsäureextraktion) bis hin zur Detektionsreaktion an einer festen Phase resultiert, dass die komplexe Automatisierung einer Nukleinsäurenanalytik leicht zu realisieren und somit besonders vorteilhaft ist.

Das verwendete Trägermaterial ist dadurch gekennzeichnet, dass sich auf der Oberfläche mindestens eine kovalent gebundene Schicht befindet, die mindestens zwei verschiedene funktionelle Gruppen trägt, die statistisch verteilt auf der Oberfläche vorliegen, wobei
mindestens eine der funktionellen Gruppen negativ geladen ist,
mindestens eine weitere funktionelle Gruppe positiv geladen oder chemisch reaktiv ist oder beide Eigenschaften besitzt.
Unter einer statistischen Verteilung der funktionellen Gruppen versteht man im Sinne der Erfindung, dass diese Gruppen nicht räumlich voneinander isoliert vorliegen, sondern sich gleichmäßig verteilt an der Trägeroberfläche befinden. Das hat den großen Vorteil, dass alle Verfahrensschritte räumlich an einem Ort des Trägermaterials stattfinden können.

Charakteristisch für die bi- oder multifunktionelle feste Phase ist eine chemische Modifizierung der festen Phase (des Substrat- oder Trägermaterials) mit mindestens zwei unterschiedlich geladenen, meist ionischen, funktionellen Gruppen oder einer negativ oder positiv geladenen funktionellen Gruppe zusammen mit einer chemisch reaktiven Gruppe, wobei letztere es ermöglicht, nach erfolgter Aufreinigung das Zielmolekül kovalent zu binden. Der genutzte Prozess der chemischen Modifizierung ermöglicht es, das Verhältnis der sich auf der entsprechenden Oberfläche befindlichen funktionellen Gruppen (kationische, anionische, zum Aufbau einer kovalenten Bindung befähigte) variabel einzustellen und damit exakt zu definieren. Das wichtigste Merkmal der Erfindung ist die Kombination einer negativ geladenen funktionellen Gruppe mit einer anderen, positiv geladenen und/oder chemisch reaktiven funktionellen Gruppe, die es ermöglicht, das Zielmolekül kovalent zu binden und alle an dem Zielmolekül durchzuführenden Extraktions-, Modifikations- und Detektionsschritte am gleichen Trägermaterial zu realisieren.

Die Modifizierung der Trägermaterialien wird durch die im folgenden beschriebene Behandlung erreicht.
Die Funktionalitäten auf dem erfindungsgemäßen Trägermaterial werden gleichzeitig oder nacheinander in beliebiger Reihenfolge durch eines der im folgenden beschriebenen Verfahren generiert.
1. Erzeugung der negativ geladenen funktionellen Gruppen und anschließende weitere Modifizierung mit kationischen funktionellen Gruppen (ohne thermisch oder photochemisch labile funktionelle Gruppen)
2. Gleichzeitige Erzeugung der negativ geladenen funktionellen Gruppen zusammen mit den kationischen Funktionalitäten (ohne thermisch oder photochemisch labile-Gruppen)
3. Erzeugung der negativ geladenen funktionellen Gruppen und anschließende weitere Modifizierung mit kationischen funktionellen Gruppen und sequentiell im dritten Schritt folgende Funktionalisierung mit thermisch oder photochemisch labilen funktionellen Gruppen
4. Gleichzeitige Erzeugung der negativ geladenen funktionellen Gruppen zusammen mit den kationischen Funktionalitäten und anschließende Modifizierung mit thermisch oder photochemisch labilen Gruppen
5. Gleichzeitiges Funktionalisieren mit allen (anionisch, kationisch und thermisch/photochemisch aktivierbare) funktionelle Gruppen
6. Erzeugung der negativ geladenen funktionellen Gruppen und anschließende weitere Modifizierung thermisch oder photochemisch labile funktionelle Gruppen

Das Verfahren zur Herstellung des eingesetzten Trägers wird im folgenden näher erläutert.

Zur Erzeugung der stark negativen funktionellen Gruppen wird in einer ersten Prozeßstufe ein beliebiges Trägermaterial für die Dauer von einer Minute bis zwei Stunden, vorzugsweise für eine Stunde, bei Raumtemperatur in eine Lösung von 0,1 g bis 500 g Ätznatron, Ätzkali oder eines anderen stark basischen Stoffes pro 1000 g eines Alkohols, vorzugsweise i-Propanol getaucht. Anschließend wird das der alkoholischen Lösung entnommene Trägermaterial so lange vorzugsweise mit Wasser gewaschen, bis die Waschlösung neutral ist, und getrocknet, vorzugsweise 30 Minuten bei 100°C.
In der sich anschließenden zweiten Prozessstufe wird der vorbehandelte Träger mit einem Photoinitiator beschichtet. Als Lösungsmittel für den Initiator kommen Ketone, Alkohole, Ester und Ether in Frage. Die Konzentration des Initiators beträgt dabei bevorzugt 0,01 g 1⁻¹ bis 0,5 g 1⁻¹.
Das vorbehandelte und mit Initiator beladene Trägermaterial wird in eine Monomerlösung getaucht, aus der Monomerlösung genommen und im feuchten Zustand belichtet. Als Monomere sind polymerisationsfähige Carbonsäure-, Sulfonsäure- und Phosphorsäure-Derivate geeignet, insbesondere die Derivate der Acrylsäure, der Methacrylsäure, der Styrolsulfonsäure und -phosphorsäure bzw. auch deren Gemische. Als Lösungsmittel für die Monomeren kommen Alkohole, Ketone, Ester, Ether und insbesondere Wasser sowie Gemische derselben zum Einsatz. Die Konzentration des Monomeren beträgt vorzugsweise 1 g 1⁻¹ bis 200 g 1⁻¹, besonders geeignet sind Lösungen mit einem Monomergehalt von 50 g 1⁻¹.
Als Belichtungsquellen werden vorzugsweise Quecksilberdampflampen, Quecksilberhoch- und -höchstdrucklampen, Halogenlampen, Wolframlampen und Laser mit Emissionen im Bereich der Initiatorabsorption verwendet. Die Belichtungszeit hängt von der Intensität der Strahlungsquelle ab und beträgt je nach deren Leistung einen Bruchteil einer Sekunde bis zu Stunden.
Nach der Belichtung wird das Trägennaterial gewaschen. Als Waschflüssigkeit sind insbesondere die Lösungsmittel geeignet, die für die Herstellung der Monomerlösung eingesetzt wurden. Die präparierten Träger werden abschließend getrocknet.

Zur Erzeugung von schwach negativen funktionellen Gruppen kann der erste Schritt stark verkürzt werden bzw. entfallen.
In einem folgenden dritten Schritt wird der vormodifizierte Träger wiederum mit einem Photoinitiator beschichtet. Als Lösungsmittel für den Initiator kommen Ketone, Alkohole, Ester und Ether in Frage. Die Konzentration des Initiators beträgt dabei bevorzugt 0,01 g 1⁻¹ bis 0,5 g 1⁻¹. Das vormodifizierte und mit Initiator beladene Trägermaterial wird in eine Monomerlösung getaucht, aus der Monomerlösung genommen und im feuchten Zustand belichtet. Als Monomere sind polymerisationsfähige Ammonium-, Sulfonium- und Phosphonium-Derivate geeignet, insbesondere die Derivate der Acrylsäure, der Methacrylsäure, des Styrols und -Vinylverbindungen bzw. auch deren Gemische. Als Lösungsmittel für die Monomeren kommen Alkohole, Ketone, Ester, Ether und insbesondere Wasser sowie Gemische derselben zum Einsatz. Die Konzentration des Monomeren beträgt vorzugsweise 1 g 1⁻¹ bis 200 g 1⁻¹, besonders geeignet sind Lösungen mit einem Monomergehalt von 50 g 1⁻¹.
Als Belichtungsquellen werden vorzugsweise Quecksilberdampflampen, Quecksilberhoch- und -höchstdrucklampen, Halogenlampen, Wolframlampen und Laser mit Emissionen im Bereich der Initiatorabsorption verwendet. Die Belichtungszeit hängt von der Intensität der Strahlungsquelle ab und beträgt je nach deren Leistung einen Bruchteil einer Sekunde bis zu Stunden.
Nach der Belichtung wird das Trägermaterial gewaschen. Als Waschflüssigkeit sind insbesondere die Lösungsmittel geeignet, die für die Herstellung der Monomerlösung eingesetzt wurden. Die präparierten Träger werden abschließend getrocknet.

In einer anderen Ausführungsvariante werden bei der photochemischen Modifizierung bereits Gemische von kationischen und anionischen Monomeren eingesetzt und gleichzeitig polymerisiert. Dazu wird der im ersten Schritt vorbehandelte Träger oder ein unvorbehandelter Träger mit einem Photoinitiator beschichtet. Als Lösungsmittel für den Initiator kommen Ketone, Alkohole, Ester und Ether in Frage. Die Konzentration des Initiators beträgt dabei bevorzugt 0,01 g 1⁻¹ bis 0,5 g 1⁻¹.
Das vorbehandelte oder auch das unvorbehandelte und mit Initiator beladene Trägermaterial wird in eine Monomerengemisch-Lösung getaucht, aus der Lösung genommen und im feuchten Zustand belichtet. Als Monomere sind polymerisationsfähige Carbonsäure-, Sulfonsäure- und Phosphorsäure-Derivate geeignet, insbesondere die Derivate der Acrylsäure, der Methacrylsäure, der Styrolsulfonsäure und -phosphorsäure bzw. auch deren Gemische im Gemisch mit polymerisationsfähigen Ammonium-, Sulfonium- und Phosphonium-Derivaten.
Als Monomere kommen auch bi- oder polyfunktionelle Monomere in Frage.

Als Lösungsmittel für die Monomeren kommen Alkohole, Ketone, Ester, Ether und insbesondere Wasser sowie Gemische derselben zum Einsatz. Die Konzentration des Monomeren beträgt vorzugsweise 1 g 1⁻¹ bis 200 g 1⁻¹, besonders geeignet sind Lösungen mit einem Monomergehalt von 50 g 1⁻¹.
Als Belichtungsquellen werden vorzugsweise Quecksilberdampflampen, Quecksilberhoch- und -höchstdrucklampen, Halogenlampen, Wolframlampen und Laser mit Emissionen im Bereich der Initiatorabsorption verwendet. Die Belichtungszeit hängt von der Intensität der Strahlungsquelle ab und beträgt je nach deren Leistung einen Bruchteil einer Sekunde bis zu Stunden.
Nach der Belichtung wird das Trägermaterial gewaschen. Als Waschflüssigkeit sind insbesondere die Lösungsmittel geeignet, die für die Herstellung der Monomerlösung eingesetzt wurden. Die präparierten Träger werden abschließend getrocknet.

Das Aufbringen der photolabilen oder der thermisch labilen funktionellen Gruppen erfolgt entweder als letzter Schritt nach Abschluß der vorangegangenen Funktionalisierungen mit negativen und positiven funktionellen Gruppen oder sofort nach der Modifizierung mit der negativen Funktionalität oder zusammen mit negativer oder/und positiver funktioneller Gruppe. Dazu wird das Substrat (der Träger) mit einem Photoinitiator beschichtet. Als Lösungsmittel für den Initiator kommen Ketone, Alkohole, Ester und Ether in Frage. Die Konzentration des Initiators beträgt dabei bevorzugt 0,01 g 1⁻¹ bis 0,5 g 1⁻¹. Das vorbehandelte oder auch das unvorbehandelte und mit Initiator beladene Trägermaterial wird in eine Monomerengemisch-Lösung getaucht, aus der Lösung genommen und im feuchten Zustand belichtet.
Als Monomere sind polymerisationsfähige Radikalgeneratoren (Azide, Öniumsalze, Peroxide, Nitroverbindungen, Ketone) bzw. auch deren Gemische geeignet, insbesondere aromatische Bisazide oder Benzophenonderivate, die photochemisch bei geeigneter Wellenlänge bzw. thermisch Radikale und somit eine kovalente Bindung zur bereits ionisch gebundenen Nukleinsäure erzeugen.
Als Lösungsmittel für die Monomeren kommen Alkohole, Ketone, Ester, Ether und insbesondere Wasser sowie Gemische derselben zum Einsatz. Die Konzentration des Monomeren beträgt vorzugsweise 1 g 1⁻¹ bis 200 g 1⁻¹, besonders geeignet sind Lösungen mit einem Monomergehalt von 50 g 1⁻¹.
Als Belichtungsquellen werden vorzugsweise Quecksilberdampflampen, Quecksilberhoch- und -höchstdrucklampen, Halogenlampen, Wolframlampen und Laser mit Emissionen im Bereich der Initiatorabsorption verwendet. Die Belichtungszeit hängt von der Intensität der Strahlungsquelle ab und beträgt je nach deren Leistung einen Bruchteil einer Sekunde bis zu Stunden.

Nach der Belichtung wird das Trägermaterial gewaschen. Als Waschflüssigkeit sind insbesondere die Lösungsmittel geeignet, die für die Herstellung der Monomerlösung eingesetzt wurden. Die präparierten Träger werden abschließend getrocknet.

Durch diese auf der Oberfläche befindliche Schicht, die insbesondere durch Modifizierung der Oberfläche auf dem Wege einer an der Oberfläche initiierten radikalischen Polymerisation hergestellt wird, wobei die Funktionalitäten gleichzeitig oder in beliebiger Reihenfolge erzeugt und kovalent auf der Oberfläche gebunden werden, sind die entsprechend funktionalisierten Trägermaterialien für die Verwendung zur komplexen Nukleinsäureanalytik hervorragend geeignet.

Die Herstellung des Trägermaterials wird im folgenden an einem Ausführungsbeispiel näher erläutert.

### Ausführungsbeispiel

### Membran, negativ und positiv geladen (ionisch)

### Variante a) sequentielle Modifizierung (negativ und positiv geladene Gruppen nacheinander)

Eine Polypropylenmembran im DIN A4-Format (Porengröße 0,2 µm) wird für 5 min. in eine 150 mM Lösung von Benzophenon (Photoinitiator) in Aceton getaucht und danach getrocknet. Anschließend wird die Membran in eine Glaswanne mit der Reaktionslösung, bestehend aus 20 g/l Acrylsäure in Wasser, überschichtet. Die Glaswanne wird mit einer Glasplatte (Tief-UV-Filter, λ > 310 nm) abgedeckt. Nach 10 min. erfolgt die Belichtung an einem UV-Trockner (Beltron GmbH) bei Halblast für 10 min. (10 Passagen durch die Belichtungszone). Anschließend wird die Membran mit Methanol und Wasser extrahiert. Danach wird getrocknet und gravimetrisch der Modifizierungsgrad bestimmt.

Die bereits mit negativen Gruppen funktionalisierte Membran wird für 5 min. in eine 150 mM Lösung von Benzophenon (Photoinitiator) in Aceton getaucht und danach getrocknet. Anschließend wird die Membran in eine Glaswanne mit der Reaktionslösung, bestehend aus 30 g/12-Aminoethylmethacrylsäureamid Hydrochlorid in Wasser, überschichtet.
Die Glaswanne wird mit einer Glasplatte (Tief-UV-Filter, λ > 310 nm) abgedeckt. Nach 10 min. erfolgt die Belichtung an einem UV-Trockner (Beltron GmbH) bei Halblast für 20 min. (20 Passagen durch die Belichtungszone). Anschließend wird die Membran mit Methanol und Wasser extrahiert. Danach wird getrocknet und gravimetrisch der Modifizierungsgrad bestimmt.

### Variante b) simultane Modifizierung (negativ und positiv geladene Gruppen gleichzeitig)

Eine Polypropylenmembran im DIN A4-Format (Porengröße 0,2 µm) wird für 5 min. in eine 150 mM Lösung von Benzophenon (Photoinitiator) in Aceton getaucht und danach getrocknet. Anschließend wird die Membran in eine Glaswanne mit der Reaktionslösung, bestehend aus 20 g/l Acrylsäure und 30 g/l 2-Aminoethylmethacrylsäureamid Hydrochlorid in Wasser, überschichtet. Die Glaswanne wird mit einer Glasplatte (Tief-UV-Filter, λ > 310 nm) abgedeckt. Nach 10 min. erfolgt die Belichtung an einem UV-Trockner (Beltron GmbH) bei Halblast für 20 min. (20 Passagen durch die Belichtungszone). Anschließend wird die Membran mit Methanol und Wasser extrahiert. Danach wird getrocknet und gravimetrisch der Modifizierungsgrad bestimmt.

FTIR-ATR-Spektroskopie:
1720 cm⁻¹ (C=O Valenzschwingung) Carboxyl-Gruppe
1650 cm⁻¹ (C=O Valenzschwingung) Säureamid I
1540 cm⁻¹ (N-H-Deformation) Säureamid II
3300 cm⁻¹ (N-H-Valenzschwingung) Amino-Gruppe (protoniert)

## Patentansprüche

1. Verfahren zur komplexen und automatisierbaren Analytik von Nukleinsäuren, wobei ein Trägermaterial verwendet wird, das auf seiner Oberfläche mindestens eine kovalent gebundene Schicht aufweist, welche mindestens zwei verschiedene funktionelle Gruppen trägt, die statistisch verteilt auf der Oberfläche vorliegen, wobei mindestens eine der funktionellen Gruppen negativ geladen ist und mindestens eine weitere funktionelle Gruppe positiv geladen und/oder chemisch reaktiv ist, und wobei
(a) ein die zu analysierende Nukleinsäure enthaltendes Probenmaterial mit dem Trägermaterial inkubiert wird, wobei die Nukleinsäure an der mindestens einen negativen funktionellen Gruppe des Trägermaterials gebunden wird,
(b) die zu analysierende Nukleinsäure von der negativen funktionellen Gruppe gelöst und kovalent an die positive und/oder chemisch reaktive funktionelle Gruppe des Trägermaterials gebunden wird und
(c) weitere für die Analytik notwendige Vorbereitungs- und/oder Analyseschritte gleichzeitig oder nacheinander an der an dem Trägermaterial gebundenen Nukleinsäure durchgeführt werden.

2. Verfahren nach Anspruch 1, **dadurch gekennzeichnet, dass** die Bindung der zu analysierenden Nukleinsäure an der mindestens einen negativen funktionellen Gruppe des Trägermaterials durch Inkubation des Probenmaterials und des Trägermaterials in einem Lysispuffer, gegebenenfalls in Gegenwart eines proteolytischen Enzyms, erfolgt.

3. Verfahren nach einem der vorhergehenden Ansprüche, **dadurch gekennzeichnet, dass** die Bindung der zu analysierenden Nukleinsäure an der mindestens einen negativen funktionellen Gruppe durch Zusatz eines Bindungspuffers erfolgt.

4. Verfahren nach einem der vorhergehenden Ansprüche, **dadurch gekennzeichnet, dass** nach erfolgter Bindung der Nukleinsäure an die mindestens eine negative funktionelle Gruppe das Trägermaterial mit dem an diesem gebundenen Probenmaterial gewaschen wird.

5. Verfahren nach einem der vorhergehenden Ansprüche, **dadurch gekennzeichnet, dass** die Ablösung der zu analysierenden Nukleinsäure von der negativen funktionellen Gruppe des Trägermaterials durch Zugabe eines Puffers, insbesondere eines Niedrigsalzpuffers, erfolgt.

6. Verfahren nach einem der vorhergehenden Ansprüche, **dadurch gekennzeichnet, dass** zur kovalenten Bindung der Nukleinsäure an die positive und/oder chemisch reaktive funktionelle Gruppe diese Gruppe aktiviert wird, insbesondere durch Radikalisierung.

7. Verfahren nach Anspruch 6, **dadurch gekennzeichnet, dass** die Aktivierung der positiven und/oder chemisch reaktiven funktionellen Gruppe mittels Lichteinwirkung, thermischer und/oder chemischer Behandlung erfolgt.

8. Verfahren nach einem der vorhergehenden Ansprüche, **dadurch gekennzeichnet, dass** die weiteren Analyseschritte Manipulation und/oder Detektion der an dem Trägermaterial gebundenen Nukleinsäure umfassen.

9. Verfahren nach Anspruch 8, **dadurch gekennzeichnet, dass** die Manipulation Denaturierung, chemische Modifizierung, Vervielfältigung, selektive Vervielfältigung, Hybridisierung und/oder Verdau mit Restriktionsenzymen umfasst.

10. Verfahren nach Anspruch 8 oder 9, **dadurch gekennzeichnet, dass** die an dem Trägermaterial gebundene Nukleinsäure mit spezifischen Sonden hybridisiert wird, insbesondere nach der Manipulation.

11. Verfahren nach einem der Ansprüche 8 bis 10, **dadurch gekennzeichnet, dass** die Detektion der Hybridisierung mit eingesetzten markierten Sonden indirekt enzymatisch und/oder direkt über die Markierung der Sonden erfolgt.

12. Verfahren nach einem der vorhergehenden Ansprüche, **dadurch gekennzeichnet, dass** die Analyse der Nukleinsäure die Sequenzierung, den Nachweis von Mutationen, den Nachweis von Methylierungsmustern, den Nachweis von SNP's und/oder den Nachweis von Restriktionsmustern umfasst.

## Claims

1. Method for the complex and automatable analysis of nucleic acids, wherein a carrier material is used, which provides on its surface at least one covalently-bound layer, which carries at least two different functional groups, which are present in statistical distribution on the surface, wherein at least one of the functional groups is negatively charged and at least one other functional group is positively charged and/or chemically reactive, and wherein
(a) a sample material containing the nucleic acid to be analysed is incubated with the carrier material, the nucleic acid being bound to the at least one negative functional group of the carrier material;
(b) the nucleic acid to be analysed is released from the negative functional group and bound covalently to the positive and/or chemically reactive functional group of the carrier material; and
(c) further preparatory and/or analytical stages necessary for the analysis are carried out simultaneously or successively on the nucleic acid bound to the carrier material.

2. Method according to claim 1,
**characterised in that**
the binding of the nucleic acid to be analysed to the at least one negative functional group of the carrier material takes place by incubation of the sample material and the carrier material in a lysis buffer, optionally in the presence of a proteolytic enzyme.

3. Method according to any one of the preceding claims,
**characterised in that**
the binding of the nucleic acid to be analysed to the at least one negative functional group takes place by addition of a binding buffer.

4. Method according to any one of the preceding claims,
**characterised in that**
on completion of the binding of the nucleic acid to the at least one negative functional group, the carrier material, with the sample material bound to it, is washed.

5. Method according to any one of the preceding claims,
**characterised in that**
the release of the nucleic acid to be analysed from the negative functional group of the carrier material takes place through the addition of a buffer, especially a low-salt buffer.

6. Method according to any one of the preceding claims,
**characterised in that**
to achieve the covalent binding of the nucleic acid to the positive and/or chemically reactive functional group, this group is activated, especially by radical formation.

7. Method according to claim 6,
**characterised in that**
the positive and/or chemically reactive functional group is activated by means of the effect of light, thermal and/or chemical treatment.

8. Method according to any one of the preceding claims,
**characterised in that**
the further analytical stages comprise manipulation and/or detection of the nucleic acid bound to the carrier material.

9. Method according to claim 8,
**characterised in that**
the manipulation comprises denaturing, chemical modification, duplication, selective duplication, hybridisation and/or digestion with restriction enzymes.

10. Method according to claim 8 or 9,
**characterised in that**
the nucleic acid bound to the carrier material is hybridised with specific probes, especially after the manipulation.

11. Method according to any one of claims 8 to 10,
**characterised in that**
the detection of the hybridisation takes place with inserted, marked probes, indirectly in an enzymatic manner and/or directly via the marking of the probes.

12. Method according to any one of the preceding claims,
**characterised in that**
the analysis of the nucleic acid comprises the sequencing, the detection of mutations, the detection of methylation patterns, the detection of SNPs and/or the detection of restriction patterns.

## Revendications

1. Procédé d'analyse d'acides nucléiques complexe et automatisable, dans lequel on utilise un matériau support qui porte en sa surface au moins une couche liée de manière covalente porteuse d'au moins deux groupes fonctionnels différents répartis de façon statistique sur la surface, l'un au moins des groupes fonctionnels présentant une charge négative et au moins un autre groupe fonctionnel présentant une charge positive et/ou étant chimiquement réactif, et dans lequel
(a) un matériau échantillon comprenant l'acide nucléique à analyser est incubé avec le matériau support, l'acide nucléique étant lié audit au moins un groupe fonctionnel négatif du matériau support,
(b) l'acide nucléique à analyser est séparé du groupe fonctionnel négatif et lié de manière covalente au groupe fonctionnel positif et/ou chimiquement réactif du matériau support ; et
(c) les autres étapes de préparation et/ou d'analyse nécessaires à l'analyse sont mises en oeuvre simultanément ou successivement sur l'acide nucléique lié au matériau support.

2. Procédé selon la revendication 1, **caractérisé en ce que** la liaison de l'acide nucléique à analyser audit au moins un groupe fonctionnel négatif du matériau support est réalisée par incubation du matériau échantillon et du matériau support dans un tampon de lyse, éventuellement en présence d'une enzyme protéolytique.

3. Procédé selon l'une quelconque des revendications précédentes, **caractérisé en ce que** la liaison de l'acide nucléique à analyser audit au moins un groupe fonctionnel négatif est réalisée par adjonction d'un tampon de liaison.

4. Procédé selon l'une quelconque des revendications précédentes, **caractérisé en ce que**, une fois réalisée la liaison de l'acide nucléique audit au moins un groupe fonctionnel négatif, le matériau support, et conjointement le matériau échantillon fixé sur celui-ci, sont lavés.

5. Procédé selon l'une quelconque des revendications précédentes, **caractérisé en ce que** la séparation de l'acide nucléique à analyser d'avec le groupe fonctionnel négatif du matériau support est réalisée par adjonction d'un tampon, en particulier d'un tampon ayant une faible concentration en sel.

6. Procédé selon l'une quelconque des revendications précédentes, **caractérisé en ce que** pour réaliser la liaison covalente de l'acide nucléique au groupe fonctionnel positif et/ou chimiquement réactif, ce groupe est activé, en particulier par activation radicalaire ou formation de radicaux.

7. Procédé selon la revendication 6, **caractérisé en ce que** l'activation du groupe fonctionnel positif et/ou chimiquement réactif est réalisée au moyen de lumière, d'un traitement thermique et/ou chimique.

8. Procédé selon l'une quelconque des revendications précédentes, **caractérisé en ce que** les autres étapes analytiques comprennent la manipulation et/ou la détection de l'acide nucléique lié au matériau support.

9. Procédé selon la revendication 8, **caractérisé en ce que** la manipulation comprend une dénaturation, une modification chimique, une multiplication, une multiplication sélective, une hybridation et/ou une digestion avec des enzymes de restriction.

10. Procédé selon la revendication 8 ou 9, **caractérisé en ce que** l'acide nucléique lié au matériau support est hybridé avec des sondes spécifiques, en particulier après manipulation.

11. Procédé selon l'une des revendications 8 à 10, **caractérisé en ce que** la détection de l'hybridation à l'aide de sondes marquées mises en place est réalisée indirectement par voie enzymatique et/ou directement via le marquage des sondes.

12. Procédé selon l'une quelconque des revendications précédentes, **caractérisé en ce que** l'analyse de l'acide nucléique comprend le séquençage, la mise en évidence de mutations, la mise en évidence de modèles de méthylation, la mise en évidence de SNP (polymorphisme de nucléotide unique) et/ou la mise en évidence de modèles de restriction.
